# EUROPEAN PATENT APPLICATION

(11) **EP 4 321 619 A1**
(43) Date of publication of application: **14.02.2024**
(21) Application number: 22783791.1
(22) Date of filing: 11.02.2022
(51) Int. Cl.: C12N 11/18, C12N 11/14, C12N 11/089, C12N 11/04, C12P 19/24, C12P 19/18, C12P 19/16, C12P 19/02

(54) **METHOD FOR PRODUCING TAGATOSE FROM BIOMIMETIC SILICON MINERALIZED MICROCAPSULE IMMOBILIZED MULTI-ENZYME**

(30) Priority: 07.04.2021 CN 202110370763
(71) Applicant: TIANJIN YEAHE BIOTECHNOLOGY CO., LTD, Tianjin, 300308 (CN)
(72) Inventor: MA, Yanhe, Tianjin 300308 (CN); SHI, Ting, Tianjin 300308 (CN); HAN, Pingping, Tianjin 300308 (CN)
(74) Representative: Pharma Patents International AG
(86) International application number: PCT/CN2022/076051
(87) International publication number: WO 2022/213720

(57) **Abstract**

Provided are a biomimetic silicon mineralized microcapsule immobilized multi-enzyme, a preparation method therefor, and a method for producing tagatose by using same. The preparation method comprises the following steps: (1) pre-mixing glucan phosphorylase, phosphoglucomutase, phosphoglucoisomerase, 6-phosphate tagatose 4-position epimerase and 6-phosphate tagatose phosphatase solutions, then adding the mixture to a calcium chloride solution, and then pouring same into a sodium carbonate solution, stirring and separating same to obtain calcium carbonate microspheres containing a multi-enzyme; (2) mixing the calcium carbonate microspheres with a polyethyleneimine solution to obtain polyethyleneimine-calcium carbonate microspheres after separation; (3) mixing the polyethyleneimine-calcium carbonate microspheres with a silicate solution to obtain biomimetic silicon mineralized-calcium carbonate microspheres after separation; and (4) mixing the biomimetic silicon mineralized-calcium carbonate microspheres with ethylenediamine tetraacetic acid for reaction to remove calcium carbonate, and separating same to obtain a biomimetic silicon mineralized microcapsule immobilized multi-enzyme

## Description

### Field of the Invention

The present invention relates to the field of tagatose production, and specifically relates to a method for producing tagatose with biomimetic silicon mineralized microcapsule immobilized multi-enzymes.

### Background of the Invention

Tagatose is a naturally occurring rare monosaccharide. It is a keto form of galactose and also an epimer of fructose. It has similar sweetness characteristics with sucrose, but only one-third of the calories of sucrose, and is thus called a low-calorie sweetener. It provides very fresh and pure sweetness, with a similar taste of fructose. Researches show that tagatose has important physiologically functional properties such as low calorie, low glycemic index, anti-caries effect, antioxidation, prebiotic actions, improvement of intestinal functions, immune regulation, and use as a drug precursor. It can be widely used in food, beverages, medicine, health care and other fields, having huge economic potential (Oh D-K: Tagatose: properties, applications, and biotechnological processes. App. Microbiol. Biotechnol. 2007, 76:1-8). The U.S. Food and Drug Administration (FDA) confirmed the safety of tagatose and approved it as a GRAS (Generally Regarded As Safe) product in 2001. In addition, the FDA approved it as a tooth-friendly ingredient in December 2002, and as a food additive in October 2003, which can be used as a sweetener in the food and beverage industry and in the medical or pharmaceutical field. The Joint FAO/WHO (Food and Agriculture Organization of the United Nations/World Health Organization) Expert Committee on Food Additives (JECFA) recommended tagatose as a new low-calorie sweetener which can be used as a food additive in 2001. At JECFA's 63rd meeting in 2004, it was stated that there was no need to limit the allowable daily intake (ADI) of tagatose, and the ingredient with "unspecified" ADI was assigned to the list of the safest food ingredients that can be arranged by JECFA. Korea, Australia, New Zealand and the European Union (EU) approved tagatose for marketing in 2003, 2004 and 2005 respectively, wherein EU officially approved tagatose as a novel food ingredient without any use limitations in December 2005. China also approved tagatose as a new food raw material in May 2014. At present, tagatose has been approved by more than 30 countries, as well as WHO/FAO and the Codex Alimentarius Commission, with no limitations on its allowable daily intake and usage.

Currently, a mainstream method for producing tagatose comprises steps of, for example, galactose isomerization, desalination, decolorization, separation, concentration, and crystallization to obtain pure tagatose. However, this method has several disadvantages. With this method, the galactose cannot be completely converted into tagatose, and thus the final product is a mixture of galactose and tagatose, resulting in a low conversion rate and the need of a complex tagatose separation process. While the cost for tagatose separation is high, the galactose as a raw material is not at a low price, both contributing to a high cost for tagatose production. (Rhimi M, Aghajari N, Juy M, Chouayekh H, Maguin E, Haser R, Bejar S: Rational design of Bacillus stearothermophilus US 100l-arabinose isomerase: Potential applications for d-tagatose production. Biochim. 2009, 91:650-653. Oh H-J, Kim H-J, Oh D-K: Increase in d-tagatose production rate by site-directed mutagenesis of l-arabinose isomerase from Geobacillus thermodenitrificans. Biotechnol. Lett. 2006, 28:145-149. Bosshart A, Hee CS, Bechtold M, Schirmer T, Panke S:Directed divergent evolution of a thermostable D-tagatose epimerase towards improved activity for two hexose substrates. ChemBioChem 2015,16:592-601.) Both the CJ corporation in Korea and the Tianjin Institute of Industrial Biotechnology, Chinese Academy of Sciences have developed a new route for producing tagatose with multiple enzymes *in vitro* (WO2018004310A1, CN109790524A, CN107988286A, CN109666620A, CN106399427A). With this route, starch, maltodextrin, or sucrose can be used as a raw material to produce tagatose through enzyme-based catalytic reactions in multiple steps. It represents a fundamental change from the existing isomerization-based tagatose production process. However, this new route involves multiple enzyme molecules which require a plurality of steps for extraction and purification before they can be used in the catalytic reactions, resulting in a high cost for enzyme preparation. Moreover, the biological enzymes are water-soluble molecules and thus can hardly be recovered and reused after the completion of the catalytic reactions, resulting in a waste of enzymes. These factors lead to the high production cost for the new route of tagatose production. Therefore, there is an urgent need to develop a method for producing tagatose with immobilized multi-enzymes, which can enable the immobilization of the multi-enzymes in the new route of tagatose production, so that the enzymes can be recycled and the production cost can be lowered.

Microcapsules have been widely studied as carriers for immobilizing enzymes. The hollow inner space of microcapsules allows embedment of a large number of enzyme molecules, and provides a favorable physical and chemical microenvironment for the embedded enzyme molecules, enhancing their stabilities. The semi-permeable walls of the microcapsules enable the transfer and exchange of substrates/products, thereby facilitating the progress of enzymatic reactions. Some researchers used protamine as an inducer to prepare biomimetic silicified microcapsules *in situ,* and then embedded the obtained microcapsules in calcium alginate spheres for enzyme immobilization. (Jiang Y, Sun Q, Zhang L, et al. Capsules-in-bead scaffold: a rational architecture for spatially separated multienzyme cascade system [J]. Journal of Materials Chemistry, 2009, 19(47):9068.) Other researchers used polyethyleneimine as an inducer and a silicon precursor to prepare silica particles, which were then assembled to the microcapsule walls. (Shi J, Jiang Z. An efficient and recyclable enzyme catalytic system constructed through the synergy between biomimetic mineralization and polyamine-salt aggregate assembly[J]. Journal of Materials Chemistry B, 2014, 2(28).) Although protamine can be used to prepare siliconized microcapsules through induction with a silicon precursor *in situ,* the cost of protamine is relatively expensive, which makes it not suitable for production on an industrial scale. Polyethyleneimine is an industrial reagent which has a low cost. If it can be used as an inducer to prepare silicon mineralized microcapsules for enzyme immobilization with a silicon precursor *in situ,* the production cost of the immobilized enzymes will be substantially reduced, which is beneficial to the industrial production of immobilized enzymes.

### Summary of the Invention

An object of the present invention is to provide a method for producing tagatose with biomimetic silicon mineralized microcapsule immobilized multi-enzymes. The method produces tagatose by using biomimetic silicon mineralized microcapsules as carriers for enzyme immobilization, immobilizing the multi-enzymes responsible for the enzyme-based catalytic conversions for tagatose to obtain immobilized multi-enzymes, and producing tagatose through catalysis with the immobilized multi-enzymes. With this method, the enzymes can be recycled. As the immobilized multi-enzymes can be recycled for multiple times, the amounts of enzymes required to be added in multiple cycles of tagatose production can be reduced greatly, and the production cost can thus be reduced.

The present invention uses biomimetic silicon mineralized microcapsules to co-immobilize the five major enzyme molecules involved in the preparation of tagatose: glucan phosphorylase, phosphoglucomutase, phosphoglucose isomerase, tagatose 6-phosphate 4-epimerase, and tagatose 6-phosphate phosphatase. The hollow inner space of the microcapsules allows embedment of a large number of enzyme molecules, and provides a favorable physical and chemical microenvironment for the embedded enzyme molecules, enhancing their stabilities. The semi-permeable walls of the microcapsules enable the transfer and exchange of substrates/products, thereby facilitating the progress of enzymatic reactions.

Therefore, the present invention provides biomimetic silicon mineralized microcapsule immobilized multi-enzymes for producing tagatose, which are prepared by a method comprising the following steps:
(1) pre-mixing the five enzymes involved in tagatose production: glucan phosphorylase, phosphoglucomutase, phosphoglucose isomerase, tagatose 6-phosphate 4-epimerase, and tagatose 6-phosphate phosphatase solutions, adding a solution of the above enzymes to a calcium chloride solution, pouring a sodium carbonate solution into the above solution, stirring, carrying out solid-liquid separation, and collecting a solid product, which is a calcium carbonate microsphere containing multi-enzymes;
(2) mixing the calcium carbonate microsphere containing multi-enzymes with a solution of polyethyleneimine, carrying out solid-liquid separation, and collecting a solid product, which is a polyethyleneimine-calcium carbonate microsphere;
(3) mixing the polyethyleneimine-calcium carbonate microsphere with a silicate solution, carrying out solid-liquid separation, and collecting a solid product, which is a biomimetic silicon mineralized calcium carbonate microsphere; and
(4) mixing the biomimetic silicon mineralized calcium carbonate microsphere with ethylenediamine tetraacetic acid (EDTA), allowing a reaction to remove calcium carbonate, carrying out solid-liquid separation, and collecting a solid product, which is the biomimetic silicon mineralized microcapsule immobilized multi-enzymes.

Preferably, glucan phosphorylase, phosphoglucomutase, phosphoglucose isomerase, tagatose 6-phosphate 4-epimerase, and tagatose 6-phosphate phosphatase are used in a mass ratio of (1-2) : (1-2) : (1-2) : (2-4) : (2-4).

In an embodiment, the solution of the enzymes used in step (1) comprises 0.05-0.15 mg/ml of the glucan phosphorylase, 0.05-0.15 mg/ml of the phosphoglucomutase, 0.05-0.15 mg/ml of the phosphoglucose isomerase, 0.1-0.3 mg/ml of tagatose 6-phosphate 4-epimerase, and 0.1-0.3 mg/ml of tagatose 6-phosphate phosphatase.

In a preferred embodiment, the polyethyleneimine has a concentration of 0.1-1.0 g/L and a molecular weight of 600-70,000.

Preferably, the silicate in step (3) has a concentration of 2-10 g/L. More preferably, the silicate in step (3) is sodium silicate at a concentration of 8 g/L.

In an embodiment, the operations of step (2) and step (3) are performed 1-3 times. Preferably, the operations of step (2) and step (3) are performed twice (which is the operations of step (2) and step (3) are carried out twice). This can achieve better results.

In an embodiment, the ratio of the mass of the polyethyleneimine to the mass of the calcium carbonate microsphere containing multi-enzymes is (20-50) : 1.

In an embodiment, the ratio of the mass of the silicate to the mass of the calcium carbonate microsphere containing multi-enzymes is (20-50) : 1.

In a preferred embodiment, step (1) is carried out by adding the solution of the enzymes to a 0.2-0.4 M calcium chloride solution, pouring an equal volume of sodium carbonate solution at an equal molar concentration to the calcium chloride solution under rotation at 600-1,500 r/min, allowing a reaction for 20-30 s, centrifuging at 3,000 r/min for separation, removing a supernatant, and washing with deionized water until a supernatant contains no sodium ions and chloride ions to obtain an calcium carbonate microsphere containing enzymes.

In a preferred embodiment, step (4) is carried out by using a 0.03-0.05 M EDTA solution, adjusting its pH to 5.0-6.0, mixing the EDTA solution and the microsphere obtained above in a mass ratio of (20-50) : 1 homogeneously, shaking for 10-20 min, centrifuging at 3,000 r/min for separation, removing a supernatant, washing with EDTA for 3-4 times, and washing with deionized water until a supernatant contains no EDTA to obtain the biomimetic silicon mineralized microcapsule immobilized multi-enzymes.

As a preferred embodiment of the present invention, the biomimetic silicon mineralized microcapsule immobilized multi-enzymes are prepared by a method comprising the following steps:
(1) pre-mixing the five major enzyme molecules involved in tagatose preparation, which is glucan phosphorylase, phosphoglucomutase, phosphoglucose isomerase, tagatose 6-phosphate 4-epimerase, and tagatose 6-phosphate phosphatase, in certain amounts, wherein 0.05-0.15 mg/ml of the glucan phosphorylase, 0.05-0.15 mg/ml of the phosphoglucomutase, 0.05-0.15 mg/ml of the phosphoglucose isomerase, 0.1-0.3 mg/ml of tagatose 6-phosphate 4-epimerase, and 0.1-0.3 mg/ml of tagatose 6-phosphate phosphatase are used; adding a solution of the above enzymes to a 0.2-0.4 M calcium chloride solution, pouring an equal volume of sodium carbonate solution at an equal molar concentration into the calcium chloride solution under rotation at 600-1,500 r/min, allowing a reaction for 20-30 s, centrifuging at 3,000 r/min for separation, removing a supernatant, and washing with deionized water until a supernatant contains no sodium ions and chloride ions to obtain an calcium carbonate microsphere containing enzymes;
(2) preparing a silicate solution having a concentration of 2-10 g/L, and a solution of polyethyleneimine having a molecular weight of 600-70,000 and a concentration of 0.1-1.0 g/L, mixing the solution of polyethyleneimine with the calcium carbonate microsphere containing enzymes in a mass ratio of (20-50) : 1 homogeneously for 10-20 min, centrifuging at 3,000 r/min for separation, removing a supernatant, washing with deionized water until a clear liquid contains no polyethyleneimine; adding a silicate solution to the above microsphere in a mass ratio of (20-50) : 1, mixing homogeneously for 10-20 min, centrifuging at 3,000 r/min for separation, removing a supernatant, washing with deionized water until a clear liquid contains no silicate ions; and
(3) preparing a 0.03-0.05 M EDTA solution, adjusting its pH to 5.0-6.0, mixing the EDTA solution with the microsphere obtained above in a mass ratio of (20-50) : 1 homogeneously, shaking for 10-20 min, centrifuging at 3,000 r/min for separation, removing a supernatant, washing with EDTA for 3-4 times, and washing with deionized water until a supernatant contains no EDTA to obtain the biomimetic silicon mineralized microcapsule immobilized multi-enzymes.

The multi-enzymes for producing tagatose involved in the present invention comprise glucan phosphorylase, phosphoglucomutase, phosphoglucose isomerase, tagatose 6-phosphate 4-epimerase, and tagatose 6-phosphate phosphatase. When the above five enzymes are co-immobilized in the biomimetic silicon mineralized microcapsules, the enzymes can be recycled with their catalytic efficiencies retained, thereby significantly reducing the production cost. In the present invention, the enzymes are preferably used in a ratio defined by 0.05-0.15 mg/ml of the glucan phosphorylase, 0.05-0.15 mg/ml of the phosphoglucomutase, 0.05-0.15 mg/ml of the phosphoglucose isomerase, 0.1-0.3 mg/ml of tagatose 6-phosphate 4-epimerase, and 0.1-0.3 mg/ml of tagatose 6-phosphate phosphatase, so as to ensure that desired catalytic efficiencies and recycling can be achieved when appropriated amounts of multi-enzymes are added in practical production of tagatose.

The present invention further relates to a method for producing tagatose with the immobilized multi-enzymes. The method comprises using starch or a starch derivative as a raw material, and carrying out enzyme-based catalytic conversions with the immobilized multi-enzymes to produce tagatose.

As a preferred embodiment of the present invention, the method specifically comprises taking 50-150 g/L of starch or starch derivative, an 80-120 mM HEPES buffer at pH 6.0-7.0, 10-50 mM inorganic phosphate, 3-7 mM divalent magnesium ions, 0.3-0.7 mM zinc ions or manganese ions, 3-7 U/ml of debranching enzyme, and 1-5 mg immobilized multi-enzymes/ml reaction liquid; carrying out enzyme-based catalytic conversion reactions at 40-70°C.

After the above reactions are completed, solid-liquid separation is carried out to collect the immobilized multi-enzymes, which can be recycled for the preparation of tagatose. The immobilized multi-enzymes are recycled for 1-10 times.

Compared with the prior art, the biomimetic silicon mineralized microcapsule immobilized multi-enzymes of the present invention, using the biomimetic silicon mineralized microcapsules as carriers for enzyme immobilization, has a simple process with mild conditions. In particular, the recycling of enzymes can be realized by using immobilized multi-enzymes to prepare tagatose. The recycling of enzymes greatly reduces the amounts of enzymes required to be added in multiple cycles of tagatose preparation and thereby lowers the production cost.

### Brief Description of the Drawings

Figure 1 is the conversion curve for tagatose preparation with the immobilized multi-enzymes having one biomimetic silicon mineralized layer provided by Example 1.
Figure 2 is the conversion curve for tagatose preparation with the immobilized multi-enzymes having two silicon mineralized layers provided by Example 2.
Figure 3 is the conversion curve for tagatose preparation with the immobilized multi-enzymes having three silicon mineralized layers provided by Example 3.
Figure 4 is the conversion curve for tagatose preparation with the immobilized multi-enzymes provided by Example 6, which have two silicon mineralized layers prepared with the polyethyleneimine having a molecular weight of 600.
Figure 5 is a column chart showing the recycling of the immobilized multi-enzymes of Example 6 for tagatose preparation in Example 8.

### Detailed Description of the Invention

The present invention discloses a method for producing tagatose with immobilized multi-enzymes. A person skilled in the art can, referring to the contents of the present invention, appropriately modify the parameters for implementation of the processes. Noted that all similar replacements or modifications are apparent to a person skilled in the art and are considered within the scope of the present invention. The methods of the present invention have been described through preferred embodiments. It is apparent that relevant personnel can modify or appropriately change and combine the methods and applications described herein to implement or apply the technology of the present invention without departing from the content, spirit, and scope of the present invention.

### Example 1

In this example, the biomimetic silicon mineralized microcapsule immobilized multi-enzymes were prepared by the following method.
(1) The five major enzyme molecules involved in tagatose preparation: glucan phosphorylase, phosphoglucomutase, phosphoglucose isomerase, tagatose 6-phosphate 4-epimerase, and tagatose 6-phosphate phosphatase were premixed in certain amounts, wherein 0.1 mg/ml of the glucan phosphorylase, 0.1 mg/ml of the phosphoglucomutase, 0.1 mg/ml of the phosphoglucose isomerase, 0.2 mg/ml of tagatose 6-phosphate 4-epimerase, and 0.2 mg/ml of tagatose 6-phosphate phosphatase were used. A solution of the above enzymes was added to a 0.33 M calcium chloride solution. An equal volume of sodium carbonate solution at an equal molar concentration was poured into the calcium chloride solution under rotation at 700 r/min. Reactions were allowed to carry out for 20-30 s. Then centrifugation was carried out at 3,000 r/min for separation, and a supernatant was removed. Deionized water was used for washing until a supernatant contained no sodium ions and chloride ions to obtain a calcium carbonate microsphere containing enzymes.
(2) A sodium silicate solution having a concentration of 8.5 g/L, and a solution of polyethyleneimine having a molecular weight of 1,800 and a concentration of 0.5 g/L were prepared. The solution of polyethyleneimine was mixed with the calcium carbonate microsphere containing enzymes in a mass ratio of 30 : 1 homogeneously for 10-20 min and centrifuged at 3,000 r/min for separation. After a supernatant was removed, deionized water was used for washing until a clear liquid contained no polyethyleneimine.
(3) The sodium silicate solution was added to the above microsphere in a mass ratio of 30 : 1, mixed homogeneously for 10-20 min, and centrifuged at 3,000 r/min for separation. After a supernatant was removed, deionized water was used for washing until a clear liquid contained no silicate ions to obtain a microsphere having one wall or layer.
(4) A 0.05 M EDTA solution was prepared with its pH adjusted to 5.8. The EDTA solution was mixed with the microsphere obtained above in a mass ratio of 30 : 1 homogeneously, shaken for 10-20 min, and centrifuged at 3,000 r/min for separation. After a supernatant was removed, EDTA was used for washing for 3-4 times. Then deionized water was used for washing until a supernatant contained no EDTA to obtain the biomimetic silicon mineralized microcapsule immobilized multi-enzymes.

### Example 2

Compared with Example 1, the biomimetic silicon mineralized microcapsule immobilized multi-enzymes of this example were different only in that the biomimetic silicon mineralized microcapsule had two, instead of one, walls or layers. That is, step (2) and step (3) in Example 1 were performed twice. Specifically, after the calcium carbonate microsphere containing enzymes which had one wall or layer was obtained in step (2) of Example 1, additionally, the solution of polyethyleneimine was mixed with the calcium carbonate microsphere containing enzymes which had one wall or layer in a mass ratio of 30 : 1 homogeneously for 10-20 min and centrifuged at 3,000 r/min for separation. After a supernatant was removed, deionized water was used for washing until a clear liquid contained no polyethyleneimine. The sodium silicate solution was added to the above microsphere in a mass ratio of 30 : 1, mixed homogeneously for 10-20 min, and centrifuged at 3,000 r/min for separation. After a supernatant was removed, deionized water was used for washing until a clear liquid contained no silicate ions to obtain a microsphere having two walls or layers.

### Example 3

Compared with Example 1, the biomimetic silicon mineralized microcapsule immobilized multi-enzymes of this example were different only in that the biomimetic silicon mineralized microcapsule had three, instead of one, walls or layers. That is, step (2) and step (3) in Example 1 were performed for 3 times. Specifically, after the calcium carbonate microsphere containing enzymes which had two walls or layers was obtained in Example 2 by repeating step (2) and step (3), the solution of polyethyleneimine was mixed with the calcium carbonate microsphere containing enzymes in a mass ratio of 30 : 1 homogeneously for 10-20 min and centrifuged at 3,000 r/min for separation. After a supernatant was removed, deionized water was used for washing until a clear liquid contained no polyethyleneimine. A sodium silicate solution was added to the above microsphere in a mass ratio of 30 : 1, mixed homogeneously for 10-20 min, and centrifuged at 3,000 r/min for separation. After a supernatant was removed, deionized water was used for washing until a clear liquid contained no silicate ions to obtain a microsphere having three walls or layers.

### Example 4

This example provided biomimetic silicon mineralized microcapsule immobilized multi-enzymes which were different from those in Example 1 only in that the polyethyleneimine had a molecular weight of 600 instead of 1800.

### Example 5

This example provided biomimetic silicon mineralized microcapsule immobilized multi-enzymes which were different from those in Example 1 only in that the polyethyleneimine had a molecular weight of 70,000 instead of 1800.

### Example 6

This example provided biomimetic silicon mineralized microcapsule immobilized multi-enzymes which were different from those in Example 1 only in that the polyethyleneimine had a molecular weight of 600 instead of 1800, and the biomimetic silicon mineralized microcapsule had two, instead of one, walls or layers (that is, step (2) and step (3) were performed twice).

### Comparative Example

This comparative example provided a mixture of non-immobilized multi-enzymes for producing tagatose, which had the same composition as the multi-enzyme mixture of Example 1.

### Example 7

The immobilized multi-enzymes provided by Examples 1-6, and the mixture of non-immobilized multi-enzymes provided by the Comparative Example were respectively used to prepare tagatose using the following method.

A hundred (100) g/L of starch, a 100 mM HEPES buffer at pH 6.5, 20 mM inorganic phosphate, 5 mM divalent magnesium ions, 0.5 mM zinc ions or manganese ions, 5 U/ml of debranching enzyme, 3 mg/ml of the immobilized enzymes or the mixture of non-immobilized multi-enzymes provided by any of the above Examples or the Comparative Example were/was taken. Enzyme-based catalytic conversion reactions were carried out at 70°C. The concentration of tagatose was determined by high performance liquid chromatography (HPLC).

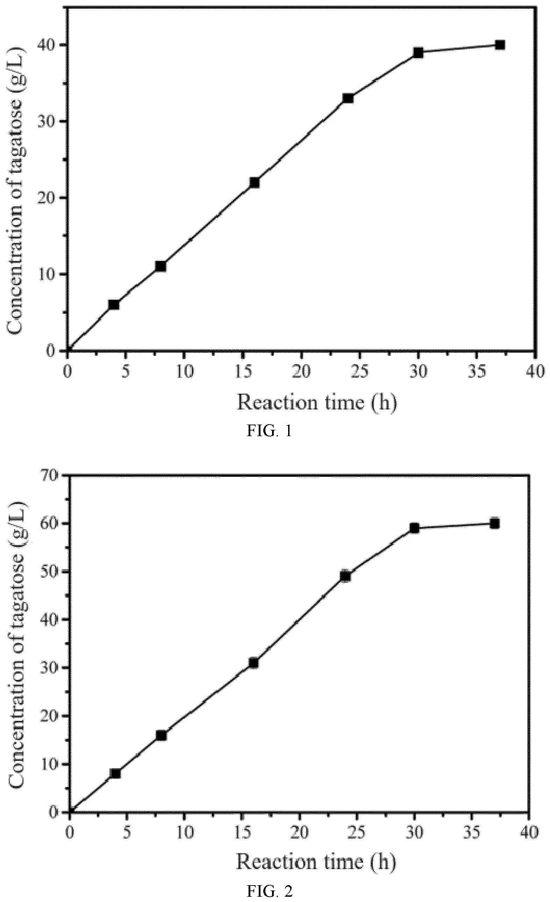

When the immobilized multi-enzymes provided by Example 1 were used for preparation, after 37 hours of reaction, the reactions were close to equilibrium, and 40 g/L of tagatose was produced with a conversion rate of 40%, as shown in Figure 1. The biomimetic silicon mineralized microcapsule of Example 1 had one wall or layer. Although the conversion rate was relatively high, the enzymes embedded in the microcapsule may leak since there was only one wall or layer. Thus, the number of wall or layer was increased based on Example 1 in an attempt to further increase the conversion rate.

When the immobilized multi-enzymes provided by Example 2 were used for preparation, after 37 hours of reaction, the reactions were close to equilibrium, and 60 g/L of tagatose was produced with a conversion rate of 60%, as shown in Figure 2. The results showed that the conversion rate was significantly improved compared with that of Example 1.

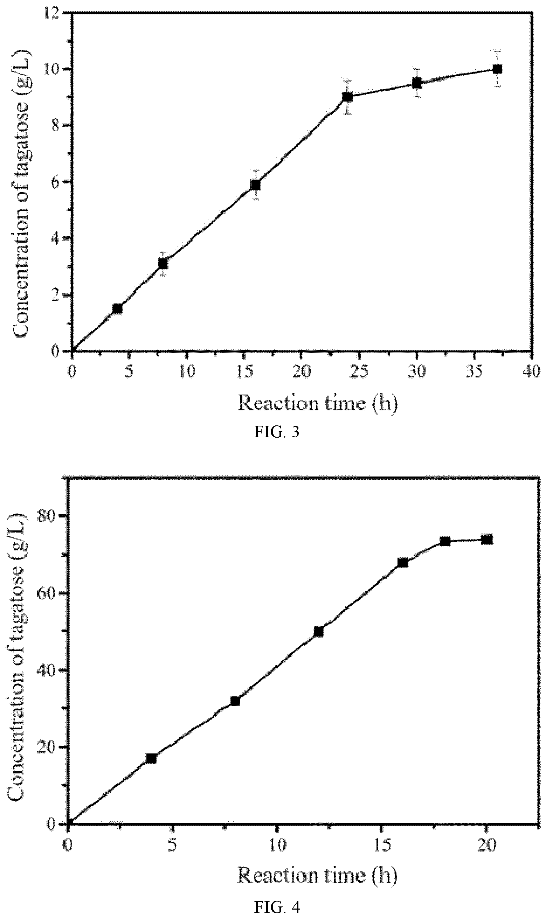

When the immobilized multi-enzymes provided by Example 3 were used for preparation, after 37 hours of reaction, the reactions were close to equilibrium, and 10 g/L of tagatose was produced with a conversion rate of 10%, as shown in Figure. 3. The biomimetic silicon mineralized microcapsule of Example 3 had three walls or layers, thicker than the two walls or layers of the biomimetic silicon mineralized microcapsule of Example 2. The increased thickness resulted in a serious mass transfer problem between the enzymes embedded in the microcapsule and the substrates outside the microcapsule. This translated into a low conversion rate for tagatose production with the immobilized multi-enzymes provided by Example 3, a rate even lower than that with the immobilized multi-enzymes provided by Example 1 which had only one layer or wall.

When the immobilized multi-enzymes provided by Example 4 were used for preparation, after 21 hours of reaction, the reactions were close to equilibrium, and 70 g/L of tagatose was produced with a conversion rate of 70%. Compared with Example 1, Example 4 used polyethyleneimine with a lower molecular weight, which induced formation of a wall having an increased thickness. Thus, although there was only one layer or wall, the leakage of the enzymes embedded in the microcapsule can be inhibited, thereby increasing the conversion rate for tagatose.

When the immobilized multi-enzymes provided by Example 5 were used for preparation, after 21 hours of reaction, the reactions were close to equilibrium, and 35 g/L of tagatose was produced with a conversion rate of 35%. Compared with Example 1, Example 5 used polyethyleneimine with a higher molecular weight, which induced formation of a wall having a reduced thickness. Due to the reduced thickness, the enzymes embedded in the microcapsule were easier to leak, thereby reducing the conversion rate for tagatose.

When the immobilized multi-enzymes provided by Example 6 were used for preparation, after 20 hours of reaction, the reactions were close to equilibrium, and 74 g/L of tagatose was produced with a conversion rate of 74%, as shown in Figure 4. Although Example 4 used polyethyleneimine with a lower molecular weight and the wall was thicker than that obtained using polyethyleneimine with a higher molecular weight, the conversion rate with only one layer was not high enough. Thus, the biomimetic silicon mineralized microcapsule of Example 6 was formed with two walls or layers, an increased compared with Example 4. The increased number of walls or layers further inhibited the leakage of the enzymes embedded in the microcapsule and thereby increased the conversion rate for tagatose.

When the mixture of non-immobilized multi-enzymes provided by the Comparative Example was used for preparation, after 10 hours of reaction, the reactions were close to equilibrium, and 72 g/L of tagatose was produced with a conversion rate of 72%.

Comparing the effects of Examples 1-6, it was found that the concentration of tagatose produced was highest when the immobilized multi-enzymes provided by Example 6 were used to prepare tagatose. Comparing the number of wall or layer of the biomimetic silicon mineralized microcapsule, it was found that when the biomimetic silicon mineralized microcapsule had two walls or layers, the effect of tagatose preparation was the best. When the microcapsule had one wall or layer, the enzymes embedded in the microcapsules may leak, thereby reducing the concentration of tagatose produced. When the microcapsule had three walls or layers, the mass transfer resistance was increased, reducing the concentration of tagatose produced.

### Example 8

### 1. Recycling of Immobilized multi-enzymes of Example 6

Tagatose was prepared with the method provided by Example 7, and then solid-liquid separation was carried out. The immobilized multi-enzymes were collected and then reused in preparation of tagatose. The concentration of tagatose produced in each cycle was determined by HPLC. The results were expressed as relative tagatose production concentration. The concentration of tagatose produced in the first cycle of reactions was set as 100%.

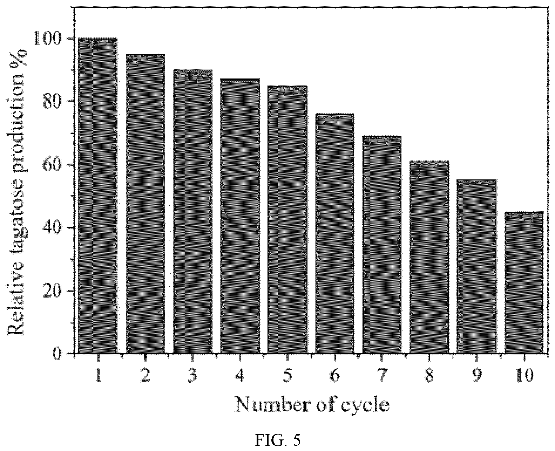

Preparation was carried out with the immobilized multi-enzymes provided by Example 6. After 10 cycles, the recycled immobilized multi-enzymes resulted in a relative tagatose production concentration of 45%, as shown in Figure 5.

### 2. Recycling of the Mixture of non-immobilized multi-enzymes of the Comparative Example

Tagatose was prepared with the method provided by the Comparative Example, and then ultrafiltration was carried out. The non-immobilized multi-enzymes were collected and reused in preparation of tagatose. After 2 cycles, the mixture of non-immobilized multi-enzymes recovered by ultrafiltration resulted in a relative tagatose production concentration of 10%.

Comparing the effects of recycling, it was found that use of immobilized multi-enzymes enabled enzyme recycling. Compared with the pure enzymes for catalytic conversions, the immobilized multi-enzymes were recycled for multiple times, which greatly reduced the amounts of enzymes required to be added in multiple cycles of tagatose preparation and lowered the production cost. When enzymes were not recycled, the pure enzymes led to a relatively high conversion rate. But when enzyme recycling was taken into consideration, the immobilized enzymes of the present invention had a great advantage over the pure enzymes.

Although the present invention has been described in detail with general description, embodiments, and experiments above, it is obvious to a person skilled in the art that some modifications or improvements can be made based on the present invention. Therefore, these modifications or improvements made without departing from the spirit of the present invention are within the claimed scope of the present invention.

## Claims

1. Biomimetic silicon mineralized microcapsule immobilized multi-enzymes for producing tagatose, **characterized in that** the immobilized multi-enzymes are prepared by a method comprising the following steps:
(1) pre-mixing the five enzymes involved in tagatose production: glucan phosphorylase, phosphoglucomutase, phosphoglucose isomerase, tagatose 6-phosphate 4-epimerase, and tagatose 6-phosphate phosphatase solutions, adding a solution of the above enzymes to a calcium chloride solution, pouring a sodium carbonate solution into the above solution, stirring, carrying out solid-liquid separation, and collecting a solid product, which is a calcium carbonate microsphere containing multi-enzymes;
(2) mixing the calcium carbonate microsphere containing multi-enzymes with a solution of polyethyleneimine, carrying out solid-liquid separation, and collecting a solid product, which is a polyethyleneimine-calcium carbonate microsphere;
(3) mixing the polyethyleneimine-calcium carbonate microsphere with a silicate solution, carrying out solid-liquid separation, and collecting a solid product, which is a biomimetic silicon mineralized calcium carbonate microsphere; and
(4) mixing the biomimetic silicon mineralized calcium carbonate microsphere with ethylenediamine tetraacetic acid (EDTA), allowing a reaction to remove calcium carbonate, carrying out solid-liquid separation, and collecting a solid product, which is the biomimetic silicon mineralized microcapsule immobilized multi-enzymes.

2. The immobilized multi-enzymes according to claim 1, **characterized in that** glucan phosphorylase, phosphoglucomutase, phosphoglucose isomerase, tagatose 6-phosphate 4-epimerase, and tagatose 6-phosphate phosphatase are used in a mass ratio of (1-2) : (1-2) : (1-2) : (2-4) : (2-4).

3. The immobilized multi-enzymes according to claim 1, **characterized in that** the solution of the enzymes used in step (1) comprises 0.05-0.15 mg/ml of the glucan phosphorylase, 0.05-0.15 mg/ml of the phosphoglucomutase, 0.05-0.15 mg/ml of the phosphoglucose isomerase, 0.1-0.3 mg/ml of tagatose 6-phosphate 4-epimerase, and 0.1-0.3 mg/ml of tagatose 6-phosphate phosphatase.

4. The immobilized multi-enzymes according to claim 1, **characterized in that** the polyethyleneimine has a concentration of 0.1-1.0 g/L and a molecular weight of 600-70,000.

5. The immobilized multi-enzymes according to claim 1, **characterized in that** the silicate in step (3) has a concentration of 2-10 g/L.

6. The immobilized multi-enzymes according to claim 1, **characterized in that** the silicate in step (3) is sodium silicate at a concentration of 8 g/L.

7. The immobilized multi-enzymes according to claim 1, **characterized in that** the operations of step (2) and step (3) are performed 1, 2 or 3 time(s).

8. The immobilized multi-enzymes according to any of claims 1-7, **characterized in that** the ratio of the mass of the polyethyleneimine to the mass of the calcium carbonate microsphere containing multi-enzymes is (20-50) : 1.

9. The immobilized multi-enzymes according to any of claims 1-7, **characterized in that** the ratio of the mass of the silicate to the mass of the calcium carbonate microsphere containing multi-enzymes is (20-50) : 1.

10. The immobilized multi-enzymes according to claim 1, **characterized in that** step (1) is carried out by adding the solution of the enzymes to a 0.2-0.4 M calcium chloride solution, pouring an equal volume of sodium carbonate solution at an equal molar concentration to the calcium chloride solution under rotation at 600-1,500 r/min, allowing a reaction for 20-30 s, centrifuging at 3,000 r/min, removing a supernatant, and washing with deionized water until a supernatant contains no sodium ions and chloride ions to obtain a calcium carbonate microsphere containing enzymes.

11. The immobilized multi-enzymes according to claim 1, **characterized in that** step (4) is carried out by using a 0.03-0.05 M EDTA solution, adjusting its pH to 5.0-6.0, mixing the EDTA solution with the microsphere obtained above in a mass ratio of (20-50) : 1 homogeneously, shaking for 10-20 min, centrifuging at 3,000 r/min for separation, removing a supernatant, washing with EDTA for 3-4 times, and washing with deionized water until a supernatant contains no EDTA to obtain the biomimetic silicon mineralized microcapsule immobilized multi-enzymes.

12. A method for producing tagatose with the immobilized multi-enzymes according to any of claims 1-11, **characterized in that** the method comprises using starch or a starch derivative as a raw material, and carrying out enzyme-based catalytic conversions with the immobilized multi-enzymes to prepared tagatose.

13. The method according to claim 12, **characterized in that** the method specifically comprises taking 50-150 g/L of starch or starch derivative, a 80-120 mM HEPES buffer at pH 6.0-7.0, 10-50 mM inorganic phosphate, 3-7 mM divalent magnesium ions, 0.3-0.7 mM zinc ions or manganese ions, 3-7 U/ml of debranching enzyme, and 1-5 mg immobilized multi-enzymes/ml reaction liquid, carrying out enzyme-based catalytic conversion reactions at 40-70°C, and collecting tagatose after the reactions are completed.
